# EUROPEAN PATENT APPLICATION

(11) **EP 2 036 445 A1**
(43) Date of publication of application: **18.03.2009**
(21) Application number: 08150062.1
(22) Date of filing: 04.01.2008
(51) Int. Cl.: A23L 1/30, A23L 2/02, A23L 1/28, A61K 31/575

(54) **Nutraceutic preparation in powder form containing free plant sterols**

(30) Priority: 05.09.2007 EP 07115753
(71) Applicant: Dietetics Pharma S.r.l., 20138 Milano (IT)
(72) Inventor: Angellotti, Carlo, 20138, MILANO (IT)
(74) Representative: Fisauli, Beatrice A. M.

(57) **Abstract**

The present invention relates to a nutraceutical preparation containing, in the same unit dose, a dried powder comprising free plant sterols in the global quantity of 0.5 to 3.0 g.

The preparation preferably also contains red yeast rice, soluble natural fibres and natural antioxidant compounds, the latter generally being carried by products deriving from fruit and/or vegetables.

The invention also relates to the process for its preparation, and the use of the preparation in foodstuffs to reduce and control the cholesterol level, mainly in the blood.

## Description

The present invention relates to a nutraceutical preparation comprising free plant sterols, and to its preparation process and the use of the preparation in the diet to reduce and control the cholesterol level, mainly in the blood.

In particular, the nutraceutical preparation according to the present invention reduces the cholesterol levels associated with low-density lipoproteins in human blood.

It is well known that cardiovascular disease is very common nowadays, and is a major cause of death, especially among the populations of Western Europe and

North America. Unfortunately, these diseases are also becoming increasingly widespread in the developing countries.

Many factors contribute to the onset of cardiovascular disease, such as a hereditary predisposition, gender, lifestyle factors such as smoking, diet, sedentary lifestyle, age, hypertension and hyperlipidaemia, which includes hypercholesterolaemia. These factors, especially the last two, contribute to the development of atherosclerotic plaques, which are a leading cause of vascular and heart disease.

There is a close relationship between high levels of cholesterol associated with low-density lipoproteins (LDL cholesterol) and an increased risk of heart and coronary disease.

Drugs, foodstuffs and diet products which induce a reduction of LDL cholesterol in the blood have already been developed for some time to reduce this risk.

It is well known that some natural plant substances reduce the plasma cholesterol level due to a reduction in the total and LDL cholesterol levels. These substances include soya lecithin and plant sterols, for example.

Products containing these natural plant substances are already well known. The only products marketed to date which contain plant sterols that produce said effects are foodstuffs such as yoghurt.

The preparations so far known and marketed present a number of drawbacks.

It is well known that soya lecithin is far less effective than certain plant sterols in combating moderate hypercholesterolaemia, and in particular in reducing the LDL cholesterol level in the plasma.

Products containing plant sterols are therefore preferred to products containing soya lecithin, because they are much more effective. However, said foodstuffs present some disadvantages which limit their use.

First of all, in view of its nature, yoghurt has a short shelf life of only a few days, and also requires low storage temperatures. This requires the provision of suitable (refrigerated) structures for its transport and display to the public. These structures involve higher running costs than preparations which can be stored at ambient temperature.

Moreover, the daily dose of plant sterols which needs to be taken is contained in nearly 100 g of yoghurt. The yoghurt bottles therefore take up a certain amount of space.

In addition, in order to take said daily dose of plant sterols, consumers must eat a food with an energy content of 364 kJ (that is, 87 kcal) which, though not extremely high, is by no means negligible. Finally, this food contains fats, including saturated fats, which must be taken into account in the daily diet balance of persons following a low-fat and/or low-cholesterol diet.

A nutraceutical preparation which does not present the said drawbacks, or presents them to a much lesser extent, is therefore required.

A nutraceutical preparation which contains little or no fat, comprising plant sterols with a cholesterol-reducing action, has now surprisingly been found. The plant sterols present in the nutraceutical preparation according to the invention are in powder form. This means that there is no need to add a significant quantity of fat to the nutraceutical preparation in order to carry and absorb the plant sterols.

One advantage of the nutraceutical preparation according to the invention is therefore that the dietary fat intake, and consequently the global calorie intake, is reduced. This is especially advantageous for persons following a low-calorie and, if necessary, a low-fat diet, which is not unusual in individuals suffering from hypercholesterolaemia.

Another advantage is that the almost total absence of fats and micro-organisms, as in yoghurt, means that the nutraceutical preparation according to the invention does not require low storage temperatures, thus considerably increasing its shelf life, which is 2 years, and reducing the management costs of the product.

A further financial advantage derives from the considerable reduction in both volumes and weight of the nutraceutical preparation according to the invention per daily dose unit of plant sterols, compared with products based on yoghurt or similar liquid products in general. The preparation according to the invention consists of a mixture of 4-5 g of powders, while the volume and weight of fluid products is much greater.

This invention therefore relates to a nutraceutical preparation comprising, in the same unit dose, a dried power comprising free plant sterols in the global quantity of 0.5 to 3.0 g, preferably 1.0 to 3.0 g, said plant sterols comprising (percentage in weight):
1) 45-80%, preferably 55-80%, of β-sitosterol, and
2) 8-40%, preferably 10-35%, of campesterol.
β -sitosterol and campesterol constitute 70 to 100%, preferably 75 to 100%, and more preferably 80 to 100% in weight of the total plant sterol content in the nutraceutical preparation according to the present invention.

In this description, the term "sterols" includes both sterols properly so called and derivatives thereof in reduced form called "stanols".

In the present description, the term "free" referring to plant sterols means that the hydroxyl group of the sterols is free, and consequently, for example, the sterols are not in esterified form.

In addition to said sterols, other types of plant sterols can also be included, which do not exceed a total of 30%, preferably 25%, and more preferably 20% of the total weight of the plant sterols.

Specific examples of said further sterols are stigmasterol and brassicasterol.

Specific examples of stanols are sitostanol and campestanol, for example.

The nutraceutical preparation according to the present invention comprises further constituents which act independently, simultaneously and/or in synergy with the said free plant sterols, thus increasing the anticholesterol effect of the nutraceutical preparation according to the present invention.

Said preparation therefore comprises other constituents with said cholesterol-reducing effect, such as "red yeast rice". Said product has been known for a very long time for its cholesterol-lowering capacity and in general for its ability to maintain the serum lipids at optimum levels.

In the present description, "red yeast rice" means the product obtained from fermentation of red rice with various strains of Monascus yeast. Said rice has a monacolin content which is generally equal to or greater than 0.4%. In said preparation, red yeast rice with a monacolin content of 0.4% is present in the quantity of between 200, preferably 250, and 350 mg.

It is obvious that if the red yeast rice contains a monacolin content different from the percentage indicated above, the quantity of dried extract present in the preparation will obviously be varied accordingly, so that the quantity of monacolin in the preparation is between 1 and 3 mg.

Said red yeast rice is a well-known commercial product; it is generally marketed and used in the form of a powdered dried extract of red rice fermented by one or more strains of Monascus yeast. The strain most commonly used is *Monascus purpureus.* Other strains which could be used are, for example, *Monascus ruber*, *Monascus Fuliginosus, Monascus Pilosus* and *Monascus Albidus.*

In this description, "unit dose" means a single dose which can be taken by a person and can be rapidly handled and packaged, while remaining a chemically and physically stable unit dose, which includes the active constituents according to the present invention.

The nutraceutical preparation according to the present invention preferably also includes soluble plant fibres which are known to reduce the plasma levels of total and LDL cholesterol. Examples of said fibres are β-glucans, psyllium and pectin; β-glucans are preferred.

Said plant fibres are present in the preparation according to the present invention in the quantity of approximately 5 to 50 mg, and preferably 5 to 30 mg. It is now known that the consumption of β-glucans in association with fruit performs a cholesterol-lowering action, helping to reduce the plasma concentration of total and LDL cholesterol.

It is also known that antioxidant compounds possess cholesterol-lowering effects because they help to reduce the plasma levels of LDL cholesterol.

Natural antioxidant compounds include some vitamins, especially vitamin A and vitamin C, and their precursors, and other natural antioxidants such as polyphenols, flavonoids, etc.

The nutraceutical preparation according to the present invention also preferably includes, in the same unit dose, products deriving from fruit and/or vegetable processing which supply said natural antioxidant compounds and contribute to the cholesterol-reducing activity of the soluble fibres.

Said products deriving from fruit and/or vegetable processing are preferably used in wholefood form. For example, said products based on fruit and/or vegetables are dehydrated powders generally obtained from extracts, juice, centrifugate, concentrated juice, concentrated flesh, or concentrated purée.

Said products deriving from fruit and vegetable processing are present in the nutraceutical preparation according to the present invention in quantities of between approximately 10 and 30% in weight of the entire unit dose.

The product deriving from fruit and/or vegetable processing present in the nutraceutical preparation according to the present invention will preferably contain a large amount of said antioxidant agents; the total antioxidant power in said products, measured in ORAC (Oxygen Radical Absorbance Capacity) units, is preferably at least equal to or greater than 2000 µmoles TE/g, for example between 3500 and 7000 µmoles TE/g.

Products deriving from fruit and vegetables containing large amounts of natural antioxidants, such as citrus fruit, in particular oranges, soft fruits, black grapes and cabbage, are preferred.

The products deriving from fruit and/or vegetable processing used in the present invention are well-known commercial products.

The nutraceutical preparation according to the invention could include other compounds as well as said constituents, especially carbohydrate and protein nutrient compounds and small amounts of lipids.

The nutraceutical preparation according to the invention can be formulated according to any known technique for preparations in solid form; for example, it can be formulated as a single mixture of powders or an association of powders, e.g. as a kit containing two or more separate powders, or as a mixture of powders in a unit dose (sachet), tablet or capsule. The preferred formulation is a single mixture of powders (sachets with a unit dose).

The techniques used to produce said formulations are widely known.

For example, the preparation according to the present invention in the form of a single mixture of powders is manufactured with known mixing techniques in a suitable appliance, such as a V mixer, under the usual conditions.

The constituents are introduced into the appliance in any order.

Mixing is conducted at around ambient temperature or lower, generally between 10° and 30°C, and preferably between 15° and 26°C. The mixing time is usually 15 to 25 minutes.

Said nutraceutical preparation according to the invention can include any other artificial, or preferably natural, constituent habitually used by one skilled in the art in the formulation of nutraceutical preparations such as additives, excipients, preservatives, sweeteners, essences, colorants and possibly other substances. The nutraceutical preparation according to the present invention contains no probiotic enzymes.

The nutraceutical preparation according to the present invention should be taken in the approximate amount of one or two unit doses a day, preferably immediately after a meal containing fats.

A non-limiting example of the invention is set out below.

### EXAMPLE

A mixture of powders with a total weight of 4290 mg is prepared in a mixer at ambient temperature by introducing the following constituents:
1) 1400 mg of a composition in powder form comprising 980 mg of the following phytosterols:
   - 690.9 mg (70.5%) of β-sitosterol
   - 144.1 mg (14.7%) of campesterol,
   - 27.4 mg (2.8%) of brassicasterol,
   - 9.8 mg (1%) of stigmasterol,
   - 79.4 mg (8.1 %) of sitostanol,
   - 8.8 mg (0.9%) of campestanol, and
   - 19.6 mg (2%) of other sterols,
2) 260 mg of dried extract of red yeast rice with a monacolin K content of 0.4% in weight,
3) 10 mg of β-glucans
4) 1000 mg of granulated orange juice powder,
5) 1000 mg of fructose,
6) 600 mg of a mixture of fruit and vegetable extracts,
7) 20 mg of silicon dioxide, Aerosil®

The dried extract of red yeast rice used is a powder with a monacolin content determined by HPLC.

The said mixture of fruit and vegetable extracts is sold as a powder. The mixture contains extracts of green tea leaves, pine bark, tomato, carrot, spinach, kale and Brussels sprouts. Said mixture has an oxidising power, expressed in ORAC units, of 5000 µmoles TE/g.

The granular orange juice powder contains 11% in weight of dehydrated orange juice.

Mixing is conducted at a temperature of approximately 25°C, and continues for approximately 15-25 minutes.

The mixture of powders thus obtained constitutes a unit dose.

Around 10 days' treatment with two unit doses a day in a patient suffering from moderate hypercholesterolaemia reduces the plasma LDL cholesterol level by approximately 10%.

## Claims

1. A nutraceutical preparation comprising a dried power comprising free plant sterols in the global quantity of 0.5 to 3.0 g, said plant sterols comprising (percentage in weight):
1) 45-80% of β-sitosterol and
2) 8-40% of campesterol;
said β-sitosterol and campesterol constitute between 70 and 100% of the total weight content of the plant sterols in the preparation.

2. The nutraceutical preparation according to claim 1, wherein the preparation comprises free plant sterols in the global quantity of 1.0 to 3.0 g.

3. The nutraceutical preparation according to claim 1 or 2, wherein the preparation comprises (percentage in weight):
1) 55-80% of β-sitosterol and
2) 10-35% of campesterol.

4. The nutraceutical preparation according to claims 1 to 3, wherein the preparation comprises 75 to 100% in weight of β-sitosterol and campesterol as a percentage of the total weight content of the plant sterols in the preparation.

5. The nutraceutical preparation according to claims 1 to 4, wherein the preparation comprises 80 to 100% in weight of β-sitosterol and campesterol as a percentage of the total weight content of the plant sterols in the preparation.

6. The nutraceutical preparation according to claims 1 to 5, wherein the preparation comprises 250 to 350 mg of a dried extract of red rice fermented by at least one strain of Monascus yeast, said extract having a monacolin content of 0.4%.

7. The nutraceutical preparation according to claims 1 to 6, comprising 5 to 50 mg of soluble plant fibres in the same unit dose.

8. The nutraceutical preparation according to claim 7, wherein the soluble plant fibres are chosen from among β-glucans, psyllium and pectin.

9. The nutraceutical preparation according to claims 1 to 8, also including a product deriving from fruit and/or vegetable processing.

10. The nutraceutical preparation according to claim 9, wherein the product deriving from fruit and/or vegetable processing is a dehydrated powder in the quantity of 10 to 30% in weight of the entire unit dose.

11. The nutraceutical preparation according to claims 9 and 10, wherein the product derives from citrus fruit processing.

12. The nutraceutical preparation according to claims 9 to 11, wherein the product deriving from fruit and/or vegetable processing is a wholefood product.

13. The nutraceutical preparation according to claims 9 to 12, wherein the product deriving from fruit and/or vegetable processing has an antioxidant power greater than 2000 µmoles TE/g.

14. The nutraceutical preparation according to claims 1 to 13, formulated according to a formulation chosen from among a mixture of powders and an association of powders.

15. The nutraceutical preparation according to claim 14, formulated as a powder mixture in a unit dose (sachet), tablet or capsule.

16. A process for manufacturing the nutraceutical preparation according to claims 1 to 15, formulated as a powder mixture which involves mixing the sterols with the dried extract of red yeast rice.

17. The process according to claim 16, wherein the soluble plant fibres and the product deriving from fruit and/or vegetable processing are also mixed with the sterols.

18. The nutraceutical preparation according to claims 1 to 15, for the treatment of hypercholesterolaemia.
